# EUROPEAN PATENT APPLICATION

(11) **EP 1 479 774 A1**
(43) Date of publication of application: **24.11.2004**
(21) Application number: 03076521.8
(22) Date of filing: 20.05.2003
(51) Int. Cl.: C12N 15/48, A61K 39/21

(54) **Viruses dependent on inducing agents for viral entry into a host cell**

(71) Applicant: Academisch Medisch Centrum, 1105 AZ Amsterdam (NL)
(72) Inventor: Baldwin, Christopher Eric, 1098 GP Amsterdam (NL); Berkhout, Benjamin, 1412 GH Naarden (NL)
(74) Representative: Prins, Adrianus Willem, Mr. Ir.

(57) **Abstract**

The invention provides a method for rendering a virus dependent on an inducing agent for viral entry by means of a pairing of two domains (HR1 and HR2) of an envelope glycoprotein into a host cell, wherein said inducing agent is capable of interfering in said pairing, comprising increasing the affinity between a HR1 domain and a HR2 domain of said envelope glycoprotein of said virus to such extent that said pairing occurs without the presence of a host cell (a premature switch) when said inducing agent is essentially not present. Preferably, said inducing agent comprises a peptide fusion inhibitor or a chaotrophic agent. A virus dependent on an inducing agent for viral entry is also herewith provided, as well as a viral replicon comprising a nucleic acid sequence with a mutation in a region encoding a HR1 domain and/or HR2 domain of an envelope glycoprotein, resulting in an increased affinity between said HR1 domain and said HR2 domain to such extent that pairing of said HR1 and HR2 domains occurs without the presence of a host cell when an inducing agent is essentially not present. A vaccine comprising said virus and/or viral replicon is also provided.

## Description

The invention relates to the field of molecular biology and immunology. More specifically, the invention relates to methods for producing viruses and/or replicons dependent on inducing agents, and vaccines comprising such viruses and/or replicons.

Despite the success of Highly Active Antiretroviral Therapy (HAART), the emergence of drug resistant strains has become a widespread and growing problem. Furthermore, although live-attenuated virus vaccines are in principle a method of choice for eliciting potent broad-based immune responses and long-term memory for life-long protection in immunized individuals, safety concerns often remain about either the reversion of attenuated vaccine strains to virulent phenotypes or the induction of fulminant infection in (immunocompromised) individuals. For instance, HIV-1 delta 3 vaccine candidate, containing 3 deletions in non-essential parts of the genome, is able to regain full replication capacity within four months of replication in tissue culture (Berkhout et al., 1999). Moreover, replication of deletion variants of the simian immunodeficiency virus (SIV) has been reported to increase after several years in some infected monkeys, concomitant with the onset of AIDS (Baba et al., 1999).

In order to overcome the problem of reversion of attenuated virus strains to virulent phenotypes, the present inventors have developed a HIV-1 variant of which the replication depends on the addition of an inducing agent such as the non-toxic, selective effector doxycycline (WO 01/20013, owned by the present applicant, incorporated herein by reference). In these strains, at least one gene essential for replication is placed under the control of an inducible repressor/activator. These virus strains are suitable for use as a vaccine, since the amount of replication can be regulated in order to elicit a good immune response without any replication beyond that level.

It is an object of the present invention to provide further means for regulation of virulence of a virus.

While present approaches are concerned with regulation of replication of viruses, the present inventors have developed methods for interfering with the entry of a virus into a host cell (also called viral entry). The present invention provides viruses which are dependent on an inducing agent for viral entry into a host cell.

The invention is further exemplified by the preferred embodiments relating to Human Immunodeficiency Virus (HIV). However, the invention is applicable to other pathogens, in particular viral pathogens, which enter a host cell via a mechanism that is comparable to HIV. Other suitable enveloped viruses, such as for instance Ebola and Influenza, are known to a person skilled in the art. Non-limiting examples are listed in Murphy et al, 1996.

The entry process is a critical step in virus replication cycle and is mediated by envelope glycoproteins. The envelope glycoproteins are arranged on the virus particle in trimeric envelope glycoprotein complexes comprising three surface units and three transmembrane molecules anchored within the viral membrane via the transmembrane domain.

The entry process is a critical step in the HIV replication cycle and is mediated by the envelope glycoproteins gp120 and gp4l. The envelope glycoproteins are arranged on the virus particle in trimeric envelope glycoproteins, comprising three gp120 and three gp41 molecules, anchored within the viral membrane via the gp41 transmembrane domain (Doms and Trono, 2000). Binding of the surface subunit gp120 to CD4 and a co-receptor on the T-cell surface triggers conformational changes in the envelope glycoprotein complex, leading to the insertion of the hydrophobic N-terminal fusion peptide (FP) of gp41 into the target cell membrane. Subsequent changes within the gp41 ectodomain (gp41e) involve two leucine zipper-like motifs, heptad repeat 1 (HR1) and heptad repeat 2 (HR2), which are separated by a loop region containing a conserved disulfide bridge. Ultimately, HR1 and HR2 from three gp41 molecules assemble into a six-helix bundle structure, also called a triple-hairpin structure, that juxtaposes the viral membrane and the cellular membrane, resulting in membrane fusion (Bentz, 2000;Melikyan et al., 2000;Weissenhorn et al., 1997). A similar mechanism applies for all enveloped viruses.

Peptide fusion inhibitors that bind to one of the HR motifs can block this conformational switch, and thus viral entry (Eckert and Kim, 2001;Eckert *et al*., 1999;Furuta et al., 1998). For instance, the fusion inhibitor T20 (DP178, enfuvirtide, pentafuside, fuzeon™) is the most advanced drug of a new class of antivirals designed to inhibit viral entry. T20 is a synthetic 36 amino acid peptide homologous to the C-terminal region of HR2 (HxB2 amino acid positions 127 to 162 of gp41) (Wild et al., 1994a;Wild et al., 1994b). By competitively binding to HR1, T20 blocks the formation of the hairpin structure that is necessary for membrane fusion and viral entry (Wild et al., 1994b). Although T20 has shown promising results in phase II/III clinical trials in advanced patients on salvage therapy (Kilby et al., 1998;Church et al., 2002;Cohen et al., 2002;Kilby et al., 2002;Lalezari et al., 2003a;Lalezari et al., 2003b), HIV-1 variants that are resistant to this compound have started to emerge (Wei et al., 2002;Kilby *et al*., 2002). Continuous in vitro passaging of HIV-1 in the presence of increasing concentrations of T20 also resulted in the selection of resistant virus variants within 6 weeks (Rimsky et al., 1998).

The present inventors have recognized that the affinity between the HR1 and HR2 domains can be increased to such extent that pairing of said domains occurs without the presence of a host cell. This is called herein a "premature switch". The phrase "without the presence of a host cell" means herein that said virus has not yet bound to the surface of a host cell to such extent that membrane fusion of the viral membrane and the cellular membrane would be possible upon pairing of HR1 and HR2. Hence, pairing of HR1 and HR2 without the presence of a host cell does not result in cell entry. After said pairing has taken place without the presence of a host cell, viral entry is not possible anymore. A prematurely switched envelope protein has lost the ability to execute the membrane fusion process.

According to the invention, viral entry of a virus with said increased pairing of HR1 and HR2 can furthermore be rendered dependent on an inducing agent, capable of interfering in said pairing. As a result, viral entry in a host cell can only occur if said inducing agent is present. Hence, said virus has become dependent on said inducing agent.

The invention thus provides a method for rendering a virus dependent on an inducing agent for viral entry by means of a pairing of two domains (HR1 and HR2) of an envelope glycoprotein into a host cell, wherein said inducing agent is capable of interfering in said pairing, comprising increasing the affinity between a HR1 domain and a HR2 domain of said envelope glycoprotein of said virus to such extent that said pairing occurs without the presence of a host cell (a premature switch) when said inducing agent is essentially not present.

A HR1 domain and a HR2 domain are defined herein as two domains of a viral envelope glycoprotein that need to be paired in order for viral entry into a host cell to occur. A HR1 domain comprises at least one amino acid sequence that is capable of pairing with at least part of a HR2 domain. This pairing is preferably due to complementary conformational shapes of at least part of said HR1 and HR2 domains. In one embodiment said envelope glycoprotein comprises a gp41 protein of HIV. However, said envelope glycoprotein may as well comprise an envelope glycoprotein of other viruses, such as Ebola, Influenza and other viruses such as listed in Murphy et al, 1996, which viruses are known to share a very similar fusion-mechanism.

By increasing the affinity between a HR1 and a HR2 domain to such extent that said pairing occurs without the presence of a host cell is meant that the rate of pairing of said domains is so high that no significant amount of viruses is capable of reaching a host cell and binding it to such extent that successive membrane fusion can take place upon pairing of HR1 and HR2. Hence, successive membrane fusion cannot take place since pairing of HR1 and HR2 already occurred. Preferably, no measurable amount of viruses is capable of reaching and binding said host cell to such extent. More preferably, essentially no viruses are capable of reaching and binding to a host cell to such extent that they are capable of entering the host cell. Once said HR1 and HR2 domains are paired before said virus has bound to the surface of a host cell to such extent that membrane fusion can take place, a post-fusion conformation is acquired and said virus is no longer capable of entering a host cell. This is called a premature switch. According to the invention, said premature switch may already occur on the surface of virus-producing cells or on the surface of a virion.

The phrase "when said inducing agent is essentially not present" means that said inducing agent is either not present at all, or that said inducing agent is present in an amount small enough to ensure that a significant amount of virus particles is incapable of entering a host cell. Preferably, essentially no virus particles are capable of entering a host cell.

According to the invention, said inducing agent is capable of decreasing the rate of pairing between HR1 and HR2 if said inducing agent is present in sufficient amount. This can for instance be performed by competitive binding of said inducing agent to HR1 and/or HR2. This way, pairing between HR1 and HR2 is at least in part counteracted. This results in a delayed rate of pairing. Because of said delay, said virus particles have more time to reach and bind a host cell. Once they have bound a host cell to such extent that membrane fusion can take place, pairing of HR1 and HR2 results in viral entry. It has therefore become possible to regulate the amount of viral entry by regulating the amount of inducing agent present.

There are multiple ways to increase the affinity between a HR1 domain and a HR2 domain of an envelope glycoprotein of a virus.

According to the invention, an affinity between a HR1 domain and a HR2 domain of an envelope glycoprotein can for instance be increased by altering at least one amino acid residue in said HR1 domain and/or HR2 domain. This is preferably done by inducing at least one mutation in a nucleic acid of a virus encoding at least part of said HR1 domain and/or HR2 domain. Once such mutation(s) has been induced, the resulting nucleic acid can be replicated, preferably using a packaging cell, and virus particles with increased HR1-HR2 affinity can be produced. Methods for inducing a mutation are known in the art, as well as methods for generating virus particles, preferably using packaging cells. Preferably, replication deficient virus particles of the invention are produced by packaging cells comprising *in trans* viral elements necessary for viral replication and/or packaging.

In a preferred embodiment, a method of the invention is provided wherein at least one mutation is induced in a nucleic acid sequence encoding a HR1 domain and in a nucleic acid sequence encoding a HR2 domain. It has been found by the present inventors that a so called "double mutation" affecting both HR1 and HR2 is particularly suitable for increasing the affinity between HR1 and HR2 and for rendering a virus dependent on an inducing agent.

In one embodiment at least one mutation is induced in a nucleic acid of a virus encoding a GIV motif of a HR1 domain, which is a glycine-isoleucine-valine stretch (HxB2 amino acid positions 36 to 38 of HIV gp41 protein), which is highly conserved among different viral isolates. Preferably, said mutation results in a substitution of valine into glycine (herein called a GIG mutation), in a substitution of valine into tryptophan (a GIW mutation) or in a substitution of valine into alanine (a GIA mutation). A virus comprising a GIG or GIW mutation has been found to be T20 resistant and can therefore be screened for diagnostic purposes.

In another embodiment at least one mutation is induced in a nucleic acid of a virus encoding a three amino acid SNY motif of a HR2 domain. Preferably said mutation results in a substitution of asparagine into lysine (a SKY mutation). In a preferred embodiment a method of the invention is provided that comprises inducing at least one mutation in a nucleic acid of said virus encoding a GIV motif of said HR1 domain and a mutation in a nucleic acid of said virus encoding a SNY motif of said HR2 domain. Most preferably, said mutation in said GIV motif comprises a GIA mutation and said mutation in said SNY motif comprises a SKY mutation. As is shown in the examples, such double mutation is particularly suitable for providing a dependent virus.

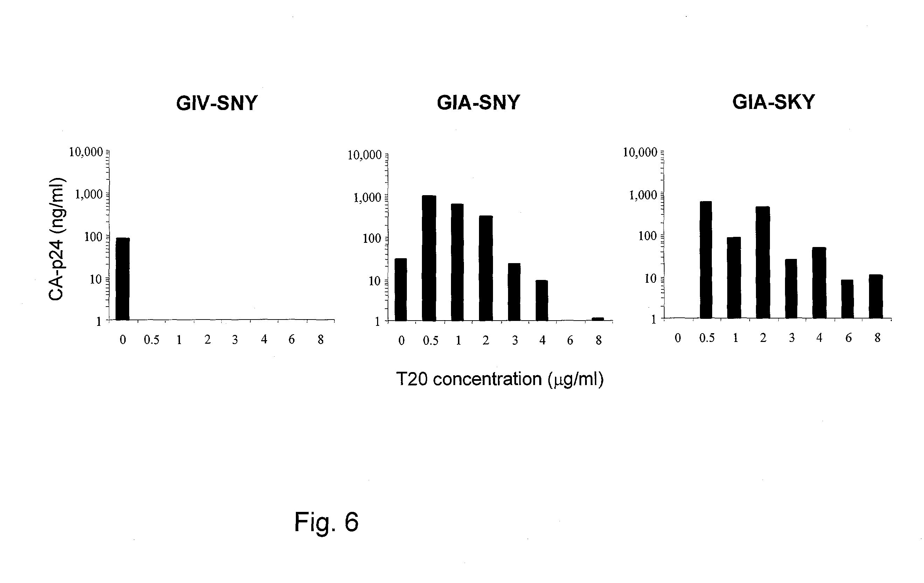

In a particularly preferred embodiment of the invention, a virus of the invention is provided that contains both a GIA mutation in a HR1 domain and a SKY mutation in a HR2 domain. Said virus (also called a GIA-SKY double mutant) has been shown to be dependent on T20 for viral entry (figure 6: no viral entry without the presence of T20).

An inducing agent capable of interfering in said pairing may be any substance capable of at least in part preventing, and/or delaying, pairing of said HR1 and HR2 domains. Said inducing agent need not be capable of completely preventing said pairing as long as said pairing is delayed long enough in order for said virus to reach and bind a host cell to such extent that membrane fusion can take place, since pairing of HR1 and HR2 at the host cell's membrane then results in viral entry.

Said inducing agent may interfere in said pairing by binding to a HR1 and/or HR2 domain, thereby for instance sterically hindering HR1-HR2 interaction. However, other ways of interfering with the pairing of two domains are possible such as and not limiting to competition for binding sites on HR1 and/or HR2 of T20 or a functional part, derivative and/or analogue thereof.

In one embodiment said inducing agent comprises a peptide fusion inhibitor or a chaotrophic agent. In a preferred embodiment said peptide fusion inhibitor comprises T20 or a functional part, derivative and/or analogue thereof. It has been shown by the present inventors that viruses dependent on T20 can be isolated, identified and/or generated. Hence, while T20 is considered to be the most advanced drug of a new class of antivirals designed to inhibit viral entry, it can surprisingly be used as well to allow for viral entry of a dependent virus of the present invention.

A functional part of T20 is defined as a part which has the same properties as T20 in kind, not necessarily in amount. For instance said functional part is as well capable of regulating viral entry of a T20-dependent virus that is provided in one embodiment of the present invention. A functional derivative of T20 is defined as a peptide which has been altered such that the properties of said molecule are essentially the same in kind, not necessarily in amount. A derivative can be provided in many ways, for instance through conservative amino acid substitution.

A person skilled in the art is well able to generate analogous compounds of T20. This can for instance be done through screening of a peptide library. Such an analogue has essentially the same properties of T20 in kind, not necessarily in amount.

A virus of the invention, dependent on an inducing agent for viral entry, obtainable by a method of the invention is also herewith provided. In one preferred embodiment said virus is a human immunodeficiency virus. A virus of the invention is particularly suitable for vaccination purposes, since it has now become possible to regulate the amount of viral entry into a host by varying the presence of an inducing agent. Hence, it has become possible to vaccinate an individual with a virus of the invention and simultaneously, and/or afterwards, administering a certain amount, or certain amounts, of inducing agent in order to regulate the amount of entry necessary for eliciting a good immune response without any viral entering of host cells beyond that level. Said virus is preferably attenuated in order to at least partly prevent harmful side effects. In a preferred embodiment a virus of the invention is provided containing a GIA mutation in a HR1 domain and a SKY mutation in a HR2 domain.

The invention also provides a viral replicon comprising a nucleic acid sequence with a mutation in a region encoding a HR1 domain and/or HR2 domain of an envelope glycoprotein, resulting in an increased affinity between said HR1 domain and said HR2 domain to such extent that pairing of said HR1 and HR2 domains occurs without the presence of a host cell when an inducing agent is essentially not present. In one embodiment, at least part of said nucleic acid sequence comprises RNA.

A replicon is defined as a nucleic acid molecule capable of replication in a suitable environment, such as a permissive cell, because it has all the necessary elements for replication in such an environment. We call it a replicon, because it will not always be directly derived from the nucleotide sequences of the original pathogen, for instance in the case of single stranded DNA viruses, RNA viruses. Typically, in order to manipulate nucleic acids, double stranded forms are necessary, typically double stranded DNA forms. Therefore preferred replicons will be double stranded DNA nucleic acids in at least one stage of their life cycle.

A replicon is also intended to reflect that the actual pathogen, or its attenuated live vaccine relative, usually comprises more than just nucleic acid. The nucleic acid is typically packaged into a (viral) particle. Therefore said replicon preferably also encodes a functional packaging signal, allowing for the nucleic acid in its wild-type-like form (RNA in the case of a retrovirus) to be packed into a viral particle. In a preferred embodiment a virus or viral replicon of the invention is provided which is derived from a human immunodeficiency virus. More preferably, said virus or viral replicon is derived from an infectious human immunodeficiency virus clone.

In a preferred embodiment, a virus of the present invention is provided of which the replication depends on the addition of an inducing agent as well. In this embodiment the safety of working with, or vaccination of, a virus of the invention is even further enhanced since both viral entry and viral replication is dependent on an inducing agent. An inducing agent allowing viral entry may be the same as an inducing agent allowing viral replication. Preferably however said inducing agent allowing viral entry is different from said inducing agent allowing viral replication. In a preferred embodiment, a virus of the invention is rendered replication-dependent on an inducing agent by a method as provided in the earlier patent application WO 01/20013 of the present applicant (incorporated herein by reference). WO 01/20013 discloses inducible viruses and viral replicons, comprising at least one inducible repressor and/or activator, and all viral sequences which are essential for replication under direct or indirect control of said inducible repressor and/or activator.

The present invention therefore provides a virus or viral replicon of the invention, further comprising at least one inducible repressor and/or activator for replication. Preferably, said inducible repressor and/or activator comprises a Tet operon or a functional equivalent thereof. In another preferred embodiment a virus or viral replicon of the invention is provided wherein at least one functional part, preferably an rtTA gene, of said inducible repressor and/or activator is inserted into the nef gene. In yet another preferred embodiment a virus or viral replicon of the invention is provided, wherein at least one NF-kb element has been deleted, and/or wherein at least one LTR is modified to avoid reversion to wild type virus.

In yet another preferred embodiment a virus or viral replicon of the invention further comprises an inactivated TAR element, an inactivated Tat element, and/or at least one tet0 motif in at least one functional LTR. Details of these preferred embodiments are outlined in WO 01/20013.

As a safety valve, it is preferred to provide a virus or viral replicon of the invention with a suicide gene that can be activated when unwanted effects occur such as replication beyond what is necessary for an immune response or rescue by wild type virus, etc. Such a suicide gene is for instance HSV-tk, which can be induced by adding gancyclovir or a functional equivalent thereof. Upon induction said gene will kill the infected cell, and thereby inhibit further replication and infection of other cells.

The invention further provides methods using a virus and/or viral replicon of the invention to produce dependent viruses, meaning viruses needing an inducing agent in order to be able to enter a host cell. Thus the invention provides a method for producing a virus dependent on an inducing agent for viral entry, comprising providing a permissive cell with a virus and/or a replicon according to the invention, culturing said cell in the presence of said inducing agent and harvesting said dependent virus from said culture. Again such methods are preferably applied to viruses such as listed in Murphy et al, 1996, more preferably applied to Ebola, Influenza and/or HIV. Thus the invention provides a method of the invention wherein said dependent virus is a human immunodeficiency virus, preferably a live-attenuated virus.

The preferred inducing agent is again a peptide fusion inhibitor or a chaotrophic agent. More preferably said inducing agent comprises T20 or a functional part, derivative and/or analogue thereof. Thus another preferred embodiment provides a method in which said inducing agent comprises a peptide fusion inhibitor or a chaotrophic agent. Yet another preferred embodiment provides a method of the invention wherein said peptide fusion inhibitor comprises T20 or a functional part, derivative and/or analogue thereof.

Also part of the present invention are viruses produced by said methods or which can be produced by said methods. Thus the invention also provides a virus dependent on an inducing agent for viral entry obtainable by a method according to the invention, preferably again a human immunodeficiency virus, again preferably live-attenuated.

A virus and/or viral replicon of the invention will find an important application in vaccination. A safe vaccine can be designed since the amount of viral entry, and, optionally, viral replication, of a virus of the invention can be regulated by an inducing agent. In one embodiment a virus of the invention is used that is dependent on T20 for viral entry and dependent on doxycycline for replication. A vaccine comprising a virus of the invention that is both dependent on an inducing agent for viral entry and dependent on an inducing agent for replication increases safety and provides novel vaccination possibilities. It is possible to induce full cycle virus replication and spread by administering both inducing agents. Alternatively, it is possible to only induce viral gene expression and immune-activation, without new infections, by administering solely said inducing agent for replication in the absence of said inducing agent for viral entry. Hence, the course of vaccination can be more precisely regulated.

The invention thus also provides a vaccine comprising a virus and/or viral replicon of the invention. Said vaccine preferably comprises an amount of inducing agent and/or a suitable adjuvant as well.

Of course said vaccine may comprise a single dosage unit, but it may also comprise the inducing agent separately, or it may be made on the spot from a replicon and/or virus that are reconstituted with a liquid excipient such as saline, optionally together with an adjuvant and/or an inducing agent. A kit of parts comprising a virus and/or viral replicon of the invention and an amount of inducing agent is therefore also herewith provided. In a preferred embodiment a kit of parts of the invention is provided wherein said inducing agent is provided separately.

Viral vaccines are well known in the field. General rules of thumb applicable to known vaccines will also apply to the vaccines of the present invention. Doses will be found through the normal dose finding studies performed during (pre)clinical trials, e.g. by simple titration of the amount of T20 as inducing agent. The vaccine may be sufficient on its own, but it may also be used in addition to other vaccines and/or therapies. The inducing agent may be needed over a longer period of time and can then be provided separately. Again the preferred vaccine is one for prophylaxis of infection with a virus as listed in Murphy et al, 1996, more preferably Ebola, Influenza, and/or human immunodeficiency virus. A vaccine or kit of parts of the invention can be used for therapeutic purposes as well, since administration to an infected individual will increase the viral load of dependent virus to induce an immune response resulting in a reduction of wild type viral load.

Regulation of the amount of inducing agent results in controlled replication of a virus or viral replicon of the invention, since at least the amount of cell entry - and, preferably, the amount of replication as well - is dependent on the amount of inducing agent(s). Regulation of the amount of inducing agent therefore results in regulation of viral replication. The invention therefore provides a method for the controlled replication of a virus or a viral replicon comprising providing a permissive cell with a virus or replicon of the invention, culturing said cell in the presence of said inducing agent and manipulating the amount of inducing agent present.

The invention also provides the use of a vaccine of the invention in that it provides a method for the prophylaxis of AIDS, comprising administering a vaccine according the invention to a patient and allowing for viral replication for a limited time by providing said inducing agent.

Booster vaccinations are possible by simple readdition of the said inducing agent at later times.

Now that a virus and a viral replicon of the invention have been provided, it is possible to improve desired characteristics. Continued replication leads to improvement of said virus and/or viral replicon by selection of spontaneous up-mutants. The beauty of working with HIV, and viruses comprising RNA in general, is that even if a poorly replicating virus is identified, the error-prone nature of the RT enzyme allows for the generation of faster-replicating variants by a method termed forced evolution (Klaver & Berkhout, 1994; Berkhout & Das, 1999). This evolutionary refinement of a virus and/or viral replicon of the invention provides a powerful method to select for fast-replicating, dependent HIV variants. Using this evolutionary approach it is possible to select for modified, improved forms of a virus and/or viral replicon of the invention. Thus, the invention also provides a method for modifying a virus or viral replicon of the invention, comprising generating a virus and/or viral replicon of the invention, providing cells, permissive for replication of said virus and/or replicon, with said virus and/or replicon, culturing said cells under conditions that allow replication of said virus and/or replicon, and obtaining replicated virus and/or replicon from said culture. Said virus and/or replicon may be derived from an infectious human immunodeficiency virus clone.

In another embodiment the invention provides a viral nucleic acid sequence comprising a mutation in a region encoding a HR1 domain and/or HR2 domain of an envelope glycoprotein resulting in an increased affinity between said HR1 domain and said HR2 domain to such extent that pairing of said HR1 and HR2 domains occurs when an inducing agent is essentially not present. Said nucleic acid may be obtained using the forced evolution approach as described above. Preferably, said inducing agent comprises a peptide fusion inhibitor or a chaotrophic agent. More preferably said inducing agent comprises T20 or a functional part, derivative and/or analogue thereof. A vector comprising a nucleic acid of the invention is also herewith provided. Such vector is particularly suitable for controlled gene therapy, allowing synchronized gene delivery.

In yet another embodiment, the present invention discloses a cell comprising a virus and/or a replicon of the invention. Said replicon may be modified by a method of the invention described in the preceding paragraph. Preferably, a cell of the invention comprises a nucleic acid sequence of the invention comprising at least one mutation in a region encoding a HR1 domain and/or HR2 domain of an envelope glycoprotein resulting in an increased affinity between said HR1 domain and said HR2 domain to such extent that pairing of said HR1 and HR2 domains occurs when an inducing agent is essentially not present.

Said cell may also be provided with a modified repressor, activator and/or promoter. In one embodiment said cell comprises a packaging cell.

It is even possible to use the enormous evolutionary capacity of an RNA virus such as HIV to select for dependent viruses of the invention with altered inducing agent-specificity, for instance by gradually changing T20 for other T20-like derivatives in the culture medium. This way, dependent variants can be selected that replicate with modified T20-molecules, such as for instance truncated or mutated T20. Preferably, viruses are selected that are dependent on smaller T20-derived peptides, preferably on T20-derived peptides of about 8-15 amino acid residues, more preferably on T20-derived peptides of about 10 amino acid residues. Said modified (preferably smaller) T20-molecules may further be used in therapy as improved antiviral drug designed to inhibit viral entry.

A (double) mutant Envelope protein of a virus of the invention will adopt a conformation that, as an immunogen, will induce antibodies that recognize intermediates of the complex fusion process, provided that said inducing agent either binds essentially irreversible to HR1 or HR2 or has significant greater affinity to said HR1 or HR2 domain. By occupying the HR1 or HR2 domain by such inducing agent, the unbound HR domain will be permanently exposed and therefore suitable to raise antibodies against. These antibodies will be able to neutralize said virus, as well as wild-type variants thereof since said wild type viruses also adopt said conformation during a fusion process. Said antibodies, or functional parts, derivatives and/or analogues thereof, are thus attractive vaccine candidates, for example 2F5-like antibodies (Zwick et al, 2001). In one aspect the invention therefore provides an isolated or recombinant proteinaceous molecule capable of specifically binding a virus of the invention. Said proteinaceous molecule preferably comprises an antibody or functional part, derivative and/or analogue thereof. A functional part of an antibody has essentially the same properties of said antibody in kind, not necessarily in amount. Said functional part is preferably capable of binding the same antigen as said antibody. However, said functional part may bind said antigen to a different extend as compared to said whole antibody. A functional part or derivative of an antibody for instance comprises a FAB fragment or a single chain antibody. An analogue of an antibody for instance comprises a chimaeric antibody.

The invention is further explained in the following examples. The examples do not limit the scope of the invention; they merely serve to exemplify the invention.

### Examples

### Methods

### HIV-1 RNA isolation, PCR amplification and sequencing

The plasma viral RNA load was measured using the Versant HIV-1 RNA 3.0 assay (bDNA) kit (Bayer Corporation, Tarrytown, NY). Total RNA was isolated from 200 µl EDTA-treated patient plasma using the Boom method (Boom et al., 1990), and eluded in a total volume of 40 µl water. Primer and template RNA (20 µl) were allowed to anneal for 2 min at room temperature in a mixture containing 4 ng of antisense primer (gp41-3'outer, 5'- GTGAGTATCCCTGCCTAACTCTAT), CMB buffer (0.01 M Tris-HCl pH 8.3, 0.05 M KCI, 0.1% Triton), 0.8 mM dNTPs and 20 units of RNasin (Promega). Upon annealing, we added 100 units of MMLV-RT (Life Technologies, Paisley, Scotland) and MgCl₂ to a final concentration of 2.4 mM, followed by incubation for 2 hours at 37°C in a total volume of 40 µl. The reaction mixture was subsequently inactivated at 95°C for 5 min. The complete reverse transcription reaction mixture (40 µl) was added to 60 µl of a PCR mixture with 100 ng of a sense primer (gp41-5'outer, 5'-GAGGGACAATTGGAGAAGTGAATT) and the antisense primer (gp41-3'outer), CMB buffer, 0.8 mM dNTPs, 1.65 mM MgCl₂ and 2 units of Taq polymerase (Perkin-Elmer Cetus). After incubation for 5 min at 95°C, the reaction mixture was subjected to 35 PCR cycles in a type 9700 DNA thermal cycler (Perkin Elmer Cetus), with each cycle including a denaturation step for 1 min at 95°C, an annealing step for 1 min at 55°C and an extension step for 2 min at 72°C. This was followed by a final extension step of 10 min at 72°C. The nested PCR was performed with 5 µl of the first PCR reaction and 50 ng sense and antisense primers (gp41-5'inner, 5'-GGAGAAGTGAATTATATAAATATAAAG, and gp41-3'inner, 5'-CACTATAGAAAGTACAGCAAAAACTAT), in a 50 µl PCR reaction as above, but with 1.2 mM MgCl₂. DNA products were analysed on a 2% agarose gel that was pre-stained with ethidium bromide.

PCR products were sequenced directly with the nested set of primers (gp41-5'inner and gp41-3'inner) using the DNA Big Dye Terminator Sequencing Kit (ABI, Foster City, CA) and an ABI 377 automated sequencer. PCR products were also cloned with the TA cloning kit (Invitrogen, San Diego, CA). White colonies were screened by PCR with primers M13 reverse and M13 (-21) provided in the TA cloning kit and the products were analysed on a 2% agarose gel for the correct size. Positive clones were sequenced with the M13 reverse and M13 (-21) primers as described above.

### Construction of LAI molecular clones

The full-length molecular HIV-1 clone pLAI (Peden et al., 1991) was used to produce wild-type and mutant viruses. The plasmid pRS1, designed to subclone mutant Env genes, was generated as follows. First, the SalI-BamHI fragment from pLAI was cloned into pUC18 (Roche, Indianapolis, IN). Second, a PstI-StuI fragment from the resulting plasmid was cloned into a pBS-SK(+)-gp160 plasmid, containing the SalI-XhoI sequences of pLAI. Mutations were introduced in pRS1 using the Quickchange mutagenesis kit (Stratagene, La Jolla, CA) and verified by DNA sequencing. Mutant Env genes in pRSl were cloned back into pLAI as SalI-BamHI fragments.

### Cell culture

The SupT1 T-cell line and the C33A cervical carcinoma cell line were maintained in RPMI 1640 medium and DMEM respectively. The transformed CEM-LuSIV cell line was maintained in RPMI 1640 medium supplemented with 300 µg/ml hygromycin B (Boehringer Mannheim, Indianapolis, IN) for maintenance of the pLuc plasmid as previously described (Roos et al., 2000). All media were supplemented with 10% fetal calf serum (FCS), penicillin and streptomycin (both at 100 units/ml). Cell cultures were incubated at 37°C with 5% CO₂.

### Transfections and CA-p24 assay

SupT1 T-cells were transfected with HIV-1 molecular clones by means of electroporation. Briefly, 5 x 10⁶ cells were washed in RPMI 1640 with 20% FCS, mixed with 1µg of DNA in 0.4-cm cuvettes, and electroporated at 250 V and 975 µF, followed by resuspension of cells in RPMI 1640 with 10% FCS. The transfected cells were split at day one post-transfection and cultured with and without T20 (Trimeris, Durham, N.C). CA-p24 production was determined from culture supernatant taken at various days post-transfection using an in-house CA-p24 antigen capture enzyme-linked immunosorbant assay (ELISA; Abbott). C33A cells were transfected by a modification of the standard calcium-phosphate protocol (Das et al., 1999) for production of viral stocks. Cell-free virus was harvested at day 3 post-transfection and stored at -80°C.

### Single cycle HIV-1 infection assay

The reporter cell line with lentiviral *tat*-driven expression ofluciferase (CEM-LuSIV cells) (Roos *et al*., 2000) were used to quantitate viral infectivity. An equal amount of virus (80-100 ng of CA-p24) was used to infect 3x10⁵ CEM-LuSIV cells in the presence or absence of T20, and 48 hours later, the cell lysate was assayed for luciferase production (Promega, Madison, Wis.), which was measured with a luminometer. The output was expressed as relative light units (RLU). To eliminate the possibility of re-infection the protease inhibitor (Saquinavir) was included in all assays.

### Drug-susceptibility analysis of the T20-resistant HIV-1 viruses

T20 susceptibility of the different T20-resistant viruses and the wild-type virus was analysed in a cell-killing assay (MTT assay) previously described (Boucher et al., 1996). Virus stocks of the different virus variants were generated as described above and used to infect SupT1 cells in the presence of increasing drug concentrations. After five to seven days, 3-(4,5-dimethylthiazol-2-yl)-2,5-diphenyltetrazolium bromide (MTT) was added to the culture. Living cells produce formazan (blue crystals) that can be quantified spectrophotometrically after solubilisation with propanol, 10% Triton X-100 and 0.4% HCl. The amount of formazan reflects the number of cells protected by the drug against killing by the virus and is used as read out for drug-susceptibility.

### Results

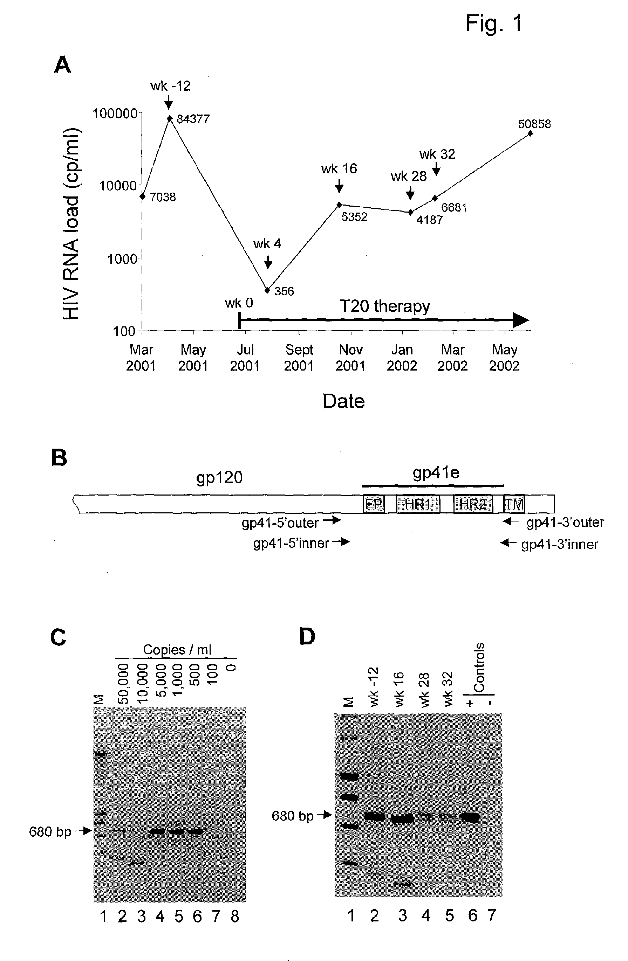

The patient involved in this study received a T20 regimen of 90 mg subcutaneous injections twice-daily for at least 32 weeks (Fig. 1A). The patient was not naive to antiviral therapy, and had previously received several drug regimens with reverse transcriptase (RT) and protease inhibitors since 1994. Prior to T20 therapy, the patient's treatment regimen failed due to the presence of a viral population with multiple resistance mutations in the RT and protease genes (results not shown). Start of T20 therapy resulted in a significant decline in HIV-1 viral RNA load from 84377 cp/ml to 356 cp/ml within 4 weeks of therapy (Fig. 1A). A rebound in viral RNA load was observed at week 16, suggesting the emergence of T20-resistant HIV-1 variants. The samples that were analysed in this study include a pre-treatment sample (week -12) and 3 samples during treatment (week 16, week 28 and week 32).

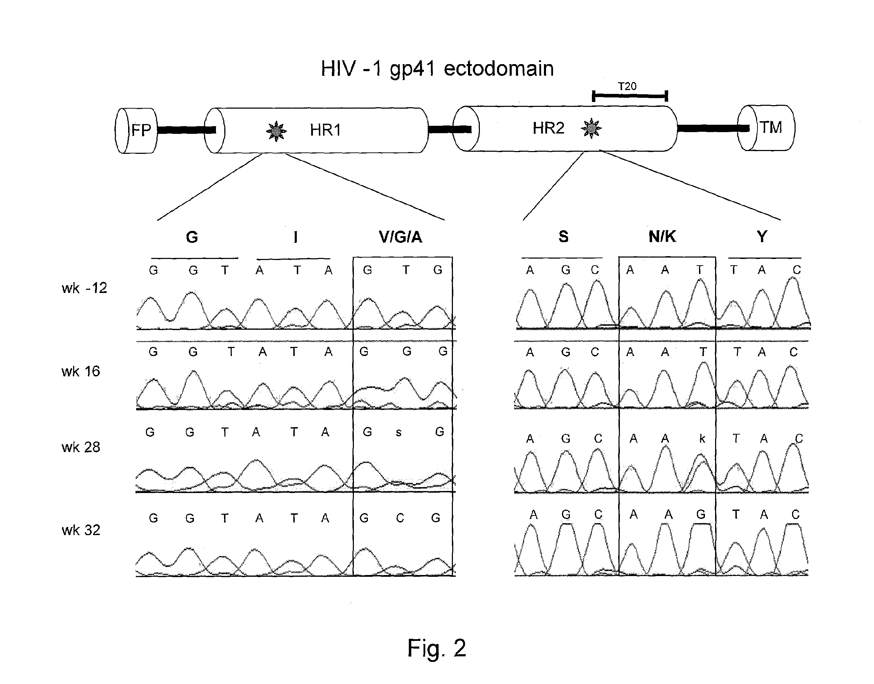

To determine whether the patient's HIV-1 population had acquired T20-resistance mutations, we performed a detailed sequence analysis of a 680 base-pair (bp) DNA fragment encoding the complete gp41e region (Fig. 1B). The viral RNA was isolated from plasma, reverse transcribed and amplified by nested PCR. We first designed a PCR protocol that is sensitive enough for the amplification of samples with a low viral load. Using a primer set based on the consensus sequence for subtype B isolates; we were able to obtain the viral sequence of the patient isolate in the sample with the highest viral load (week -12). We designed a nested primer set based on this sequence. We also varied the PCR conditions to obtain optimal MgCl₂ concentrations (2.4 mM and 1.2 mM for the first and second PCR reactions, respectively). This optimised PCR protocol is able to detect down to 500 viral RNA cp/ml (Fig. 1C), allowing the amplification of all patient samples (Fig. 1D), except for the week 4 sample (356 viral RNA cp/ml), which is under the detection limit. Direct sequence analysis of the PCR products revealed two mutational hotspots in gp41e: the GIV motif in HR1 and the SNY motif in HR2 (Fig. 2). At week 16, we observe the first codon change (gtg-to-ggg) that results in a valine to glycine substitution in HR1 (GIV to GIG). At week 28, the population sequence indicates the appearance of an additional gcg codon variant (GIA), thus yielding a complex population sequence. At the same time, we observe the appearance of an aag codon encoding lysine at amino acid position 126 in HR2 in approximately half of the population (50% SNY wild-type, 50% SKY mutant). At week 32, HIV-1 variants that combine the GIA motif in HR1 and the SKY motif in HR2 dominate the viral population.

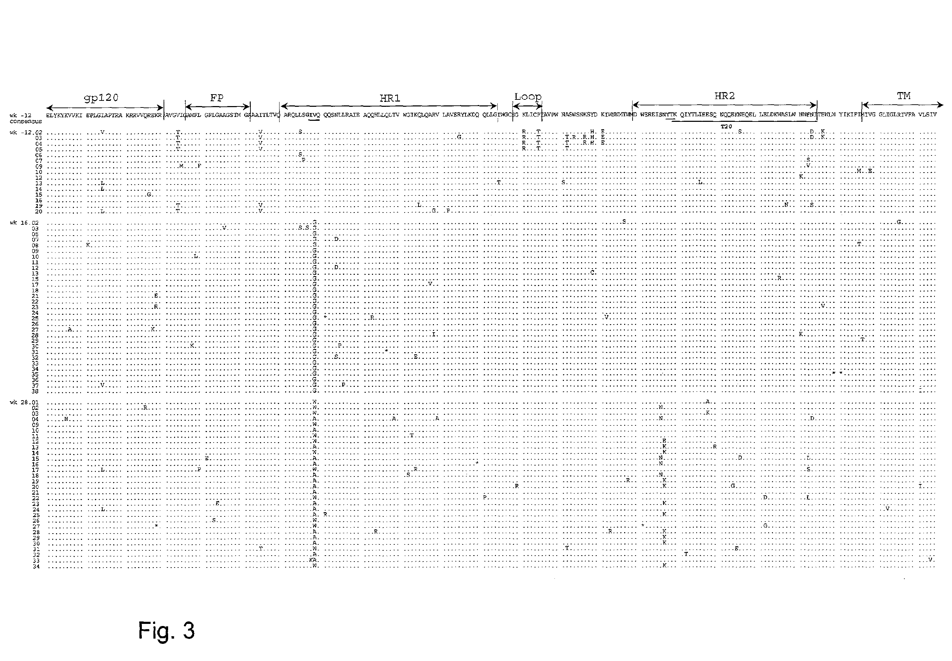

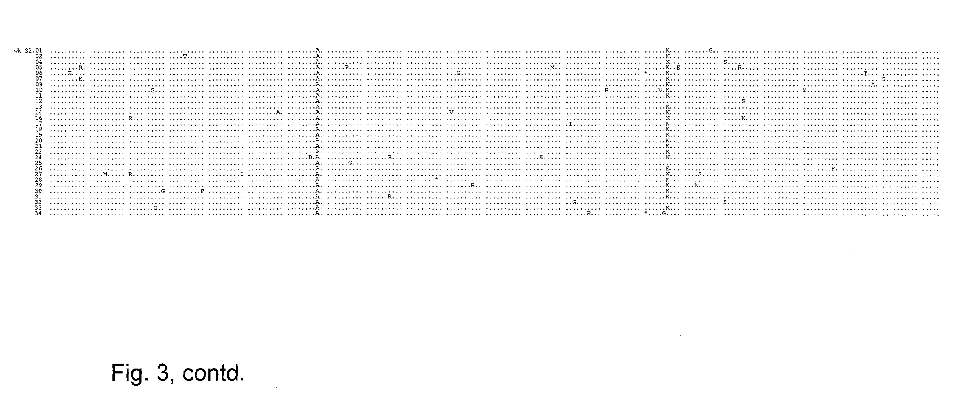

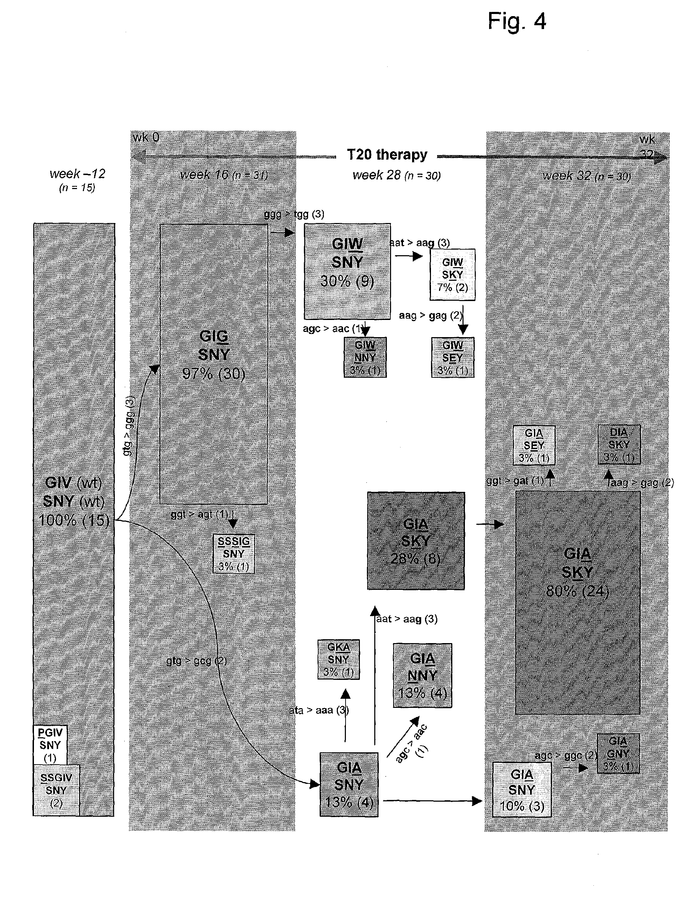

Direct sequencing of the HIV-1 quasispecies suggests a complex evolutionary pathway of the acquisition of drug-resistance mutations in both HR domains of gp41e. To obtain a more precise picture of HIV-1 evolution under T20 pressure, we performed clonal sequencing. The PCR fragments of the complete gp41e region were ligated into the TA cloning vector and multiple clones per time-point were sequenced (Fig. 3). This information allowed us to build a detailed evolutionary scheme (Fig. 4). Both the GIV and SNY motifs acquired amino acid substitutions in the course of T20 treatment, but no other changes were selected in the gp41e sequence. Analysis of the pre-treatment sample confirmed the presence of the well conserved GIV and SNY motifs in HR1 and HR2, respectively. At week 16 into T20 therapy, 97% of the viral population contained the GIG motif in HR1 (30 of 31 clones). A single clone encodes the SIG double mutant, and this viral sequence is typical because a stretch of three serine residues is created due to an additional upstream leucine to serine substitution that is already present as a minority sequence in the pre-therapy population (SSSIG compared to wild-type LSGIV). The week 28 sample shows a complex picture that is not fully predicted by the population sequence. We observed the complete disappearance of the GIG motif at the expense of GIA (16 clones), GIW (13 clones), and the GKA double mutant (1 clone). Inspection of the underlying codon changes indicates that GIW replaces the initial GIG variant, whereas GIA represents an independent evolutionary path. The week 28 sample also shows the first changes in the SNY motif of HR2. The wild-type SNY sequence is still dominant (14 clones), but the variants SKY (10 clones), NNY (5 clones), and SEY (1 clone) are also observed. Inspection of the sequences indicates that the SKY mutation in HR2 is coupled to the GIA mutation in HR1. Specifically, of the 10 SKY motifs, 8 are linked to GIA and only 2 to GIW, and the latter combines preferentially with the wild-type SNY motif (9 of 13 clones). This GIA-SKY double mutant prevails at week 32, being present in 80% of the HIV-1 sequences (24 of 30 clones). Some minor variants were also observed at weeks 28 and 32. At week 28, we observed the double mutants GIW-SKY (2 clones), GIW-SEY (1 clone) and GIW-NNY (1 clone), which are derived from the GIW-SNY intermediate by a second mutation in HR2. We also observed the GIA-NNY double mutant (4 clones), which is derived from the GIA-SNY intermediate. This GIA-NNY mutant is outcompeted by the GIA-SKY variant at week 32. This last sample also shows some minor double mutants: GIA-SEY (1 clone), DIA-SKY (1 clone) and GIA-GNY (1 clone).

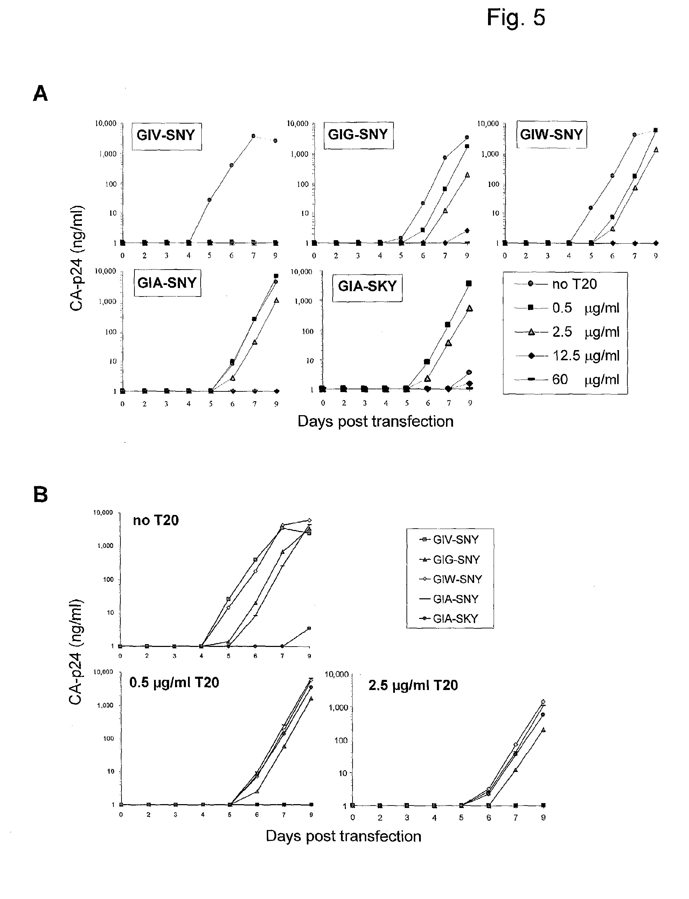

The impact of some of the major mutations observed was tested in vitro in the HIV-1 LAI molecular clone for their influence on viral replication and resistance to T20. The HIV-1 molecular clone (pLAI) was used to produce wild-type and mutant viruses containing combinations of the HR1 and HR2 mutations. We constructed a wild-type virus with the patient-derived GIV-SNY sequence in place of the GIV-NNY sequence present in the LAI molecular clone. DNA constructs were transfected into the SupTl T-cell line and cultured with and without the T20 peptide and viral replication was monitored by CA-p24 production (Fig. 5A and 5B). The wild-type virus (GIV-SNY) replicated without T20, but was strongly inhibited at all T20 concentrations tested (Fig. 5A). This is in agreement with previous studies on T20-susceptibility of commonly used laboratory isolates (Rimsky *et al*., 1998;Wild *et al*., 1994b). In contrast, viruses with mutations in HR1 (GIG-SNY, GIW-SNY and GIA-SNY) were highly resistant to T20. Surprisingly, the GIA-SKY double mutant was not only highly resistant to T20, but also critically dependent on T20 for its replication. In Figure 5B, we directly compared the replication capacity of the different viruses in the absence and at a fixed T20 concentration. In the absence of T20, the GIW-SNY virus replicated comparable to wild-type HIV-1, the GIG-SNY and GIA-SNY mutant viruses were slightly less fit and the GIA-SKY double mutant was almost fully replication-defective. In the presence of T20, the GIA-SKY virus was perfectly capable of replication, whereas the wild-type virus was not.

In another experiment (Fig. 6), we used concentrations of T20 comparable to plasma levels in patient serum receiving 100 mg twice-daily subcutaneous injections of T20 (Kilby e*t al*., 2002). We only show CA-p24 values for day 6 post-transfection which best represents the relative differences in replication capacity in the absence of T20, and the resistance / dependence phenotypes observed for the GIA-SNY and GIA-SKY mutants. As expected, the wild-type virus (GIV-SNY) replicated in the absence of T20 but was inhibited with all T20 concentrations tested (0.5 - 8 µg/ml T20). The GIA-SNY mutant was clearly resistant to the drug and showed some evidence of activation by T20. This phenotype has been observed in many independent transfections (results not shown). However, the GIA-SKY double mutant is fully dependent on T20 for its replication, and is also slightly more resistant to high T20 concentrations than the GIA-SNY single mutant, which is in agreement with our predictions.

T20 susceptibility for the wild-type and T20-resistant viruses was analysed by the MTT cell-killing assay (Boucher et al., 1996). Virus stocks of the different virus variants were used to infect SupT1 cells in the presence of increasing drug concentrations. After five to seven days, MTT reagent was added to the culture and living cells, which produce formazan (blue crystals) could be quantified spectrophotometrically. The amount of formazan reflects the number of cells protected by the drug against killing by the virus and is used as read out for drug susceptibility and IC50 calculations.

The infectivity of the virus particles was assayed with the indicator cell line CEM-LuSIV (Roos *et al*., 2000) containing the luciferase reporter gene, which is responsive to Tat protein.

### Discussion

The GIW variant is eventually taken over by a virus variant with the GIA motif. This GIA variant is observed first at week 28 and dominates the population at week 32. The eventual appearance of GIA seems coupled to the presence of a second mutation in the SNY motif of HR2. A few GIA-only clones are present at week 28, but the majority combines GIA with the mutant SKY motif. At week 32, the GIA-SKY double mutant dominates the viral quasispecies. This double mutant is likely to provide a higher level of T20-resistance than the single mutants, including the GIG, GIA and GIW variants, which are outcompeted by GIA-SKY. The SKY second site mutation is also seen in combination with GIW (2 copies at week 28), but the GIA-SKY combination seems the superior solution. These combined results can be translated in the following ranking order of replication in the presence of T20: GIV-SNY (wild-type) < GIA-SNY < GIG-SNY < GIW-SNY < GIA-SKY.

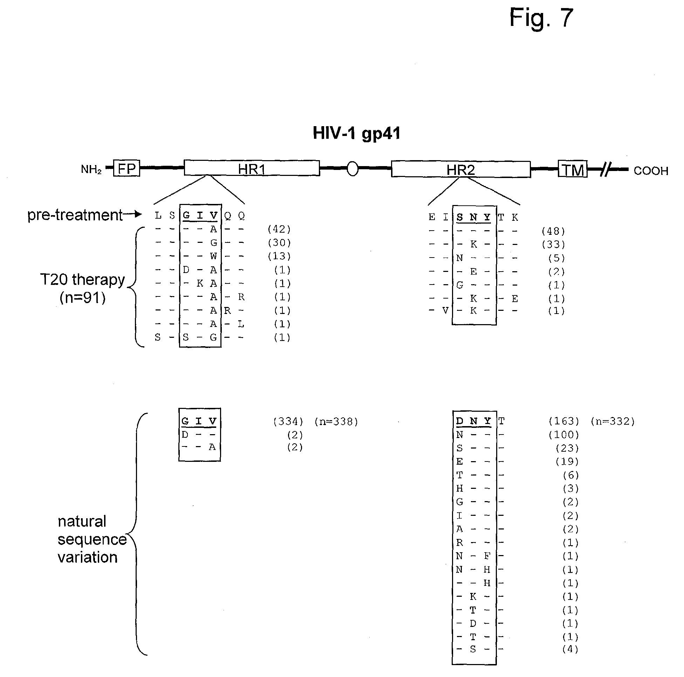

Many new gp41e mutations are reported in this study. We tabulated all observed GIV/SNY changes in Figure 7, in which we also included the sequence variation among natural isolates of HIV-1 subtype B. The GIV motif is highly conserved, with only 4 isolates among 338 sequences that deviate from this sequence (of which 2 are GIA variants). The SNY motif, in particular the first residue, is somewhat more variable (DNY, NNY, SNY and ENY represent 305 of the 332 sequences). The SKY motif that is selected under T20-therapy is never observed in natural isolates, although a single DKY variant is found. Of interest is that the asparagine (N) of the SNY motif is a site for N-linked glycosylation (Perrin et al., 1998;Johnson et al., 2001;Lee et al., 1992;Dedera et al., 1992;Dash et al., 1994). Thus, the acquisition of the SKY resistance / dependence mutation also means the loss of a glycosylation site, which is extremely unusual in natural HIV-1 strains (only 8 of 332 isolates). Strikingly, one of the two natural GIA variants in HR1 is combined with an exceptional DSY sequence in HR2. This finding relates to our observation that changes in HR2 are coupled to changes in HR1. A more extensive screening of the HIV-1 sequence database further strengthens this idea. Among 417 sequences of HIV-1 non-B subtypes, we found one additional isolate with the GIA motif, and this sequence also encodes an unusual HR2 motif (RNI) (results not shown).

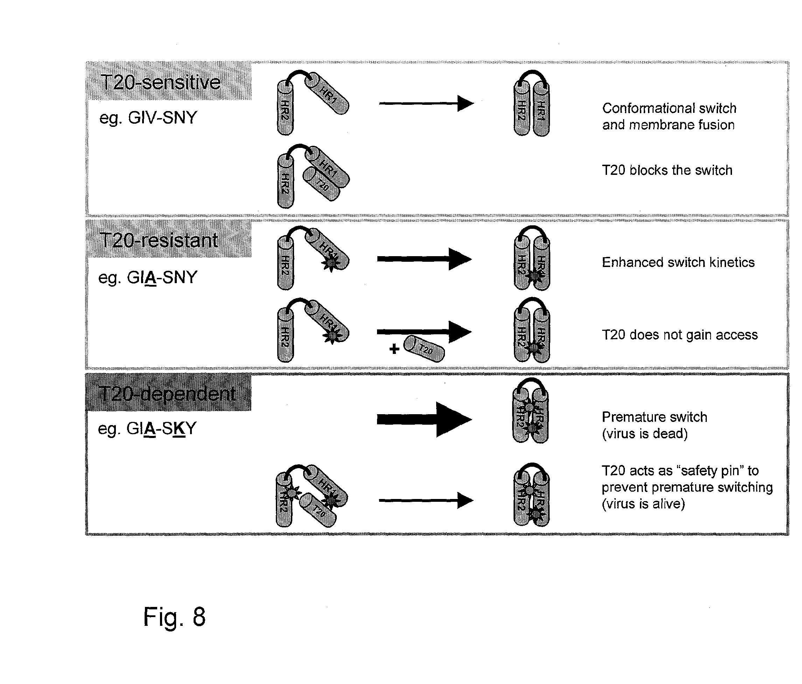

Figure 8 shows a model for T20-dependency. T20-dependence, such as for instance observed for the GIA-SKY double mutant, is due to a "premature" switch to the post-fusion conformation. Such a prematurely switched Env protein has lost the ability to execute the membrane fusion process, which explains the severe replication defect that we observe without T20. T20 can rescue this mutant by preventing the premature conformational switch. In other words, the T20 peptide acts as a "safety pin" that locks / stabilizes the pre-fusion conformation.
The T20-dependent Envelope protein of a virus of the invention is likely to adopt an unusual conformation, and it is possible that this is one of the (currently unknown) intermediates in the complex process that eventually generates the fusion-competent conformation (6-helix bundle). Thus, further elucidation of the T20-dependent mechanism provides novel, structural insight into the virus-cell fusion process, and this virus is also an attractive reagent to study the mechanism of action of other inhibitors. This insight generates new leads for the design of improved peptide-based inhibitors or other type of compounds that interfere with the fusion process.

### Brief description of the drawings

Figure. 1. **(A)** Plasma HIV-1 RNA load during T20 therapy. Plasma samples that were analysed by sequencing are marked in the graph as wk -12 (pre-therapy), wk 16, wk 28 and wk 32. T20 therapy was started at wk 0 (28-06-2001) and stopped recently at wk 50 (17-06-2002) (data not shown). At the initiation of T20 therapy, the patient also received ZDV 300 mg bid, 3TC 150 mg bid and lopinavir/r 3 caps bid. The patient's treatment regimen was modified on 24-12-2001 to TDF 300 mg QD, 3TC 150 mg bid, amprenavir 600 mg bid and ritonavir 100 mg bid. **(B)** PCR amplification scheme of gp41e. The HIV-1 envelope gene and the location of primers used to amplify the gp41e region. **(C)** Sensitivity of PCR assay. The expected DNA product of 680 bp is marked. M in lane 1 represents a DNA molecular weight marker (Smart Ladder, Promega). Lanes 2-7 contain serial dilutions of the Scott Layne viral standard (Layne et al., 1992) **(D)** PCR products obtained for the patient plasma samples (lanes 2-5). A positive control (5,8 x 10⁵ viral RNA cp/ml of the Scott Layne viral standard) and a negative control (HIV-1 negative plasma) are shown in lanes 6 and 7, respectively.
Figure. 2. Population sequence of HIV-1 gp41e under T20 therapy. The PCR products were sequenced directly. The sequence electropherograms are shown for two motifs that evolve under T20 therapy: the GIV motif in HR1 and the SNY motif in HR2. The respective codon triplets and the encoded amino acid (1 letter code) are marked on top of the graph. Standard nomenclature is used for the nucleotide code: s = G or C and k = G or T.
Figure. 3. Amino acid sequence of gp41e in individual HIV-1 clones under T20 therapy. The PCR fragments that were sequenced directly (Fig. 2) were ligated into the TA cloning vector, and 20-38 individual clones per time-point were sequenced. Only the sequences that passed the quality control are shown (full-length sequence of correct product). All clones were aligned to a consensus sequence based on the 15 sequences of the pre-treatment sample (wk -12). Dots mark amino acids that are identical to this consensus sequence. Stop codons are indicated with *. The GIV and SNY motifs are underlined and in bold. The HR2 region corresponding to the T20 peptide sequence (amino acids 127 to 162) is underlined.
Figure. 4. Evolution of T20 resistance in HIV-1 gp41e. This schematic of the viral quasispecies in the course of T20 therapy focuses exclusively on the GIV motif in HR1 and the SNY motif in HR2. The situation is depicted at week -12 (pre-therapy) and weeks 16, 28 and 32 under T20 therapy. The number of clones that were sequenced per time-point is indicated by n. The initial virus population present at week -12 consists of the fully wild-type sequence (GIV-SNY, with some upstream sequence variation such as SSGIV shown as internal boxes). The starting virus population is shown as a blue box, all single mutants are shown as hatched-coloured boxes (e.g. GIG-SNY at week 16), and the double mutants in full colour boxes (e.g. GIA-SKY at week 28 and 32). The sizes of the boxes reflect the abundance of that particular variant within the viral population. The actual percentage of that variant in the viral quasispecies and the number of clones with that sequence are indicated within the boxes. The evolution scheme was derived in part from inspection of the actual codon changes that are indicated alongside the arrows. The mutations are ranked according to their likelihood (Keulen et al., 1996;Keulen et al., 1997;Berkhout et al., 2001): the easy g-to-a and c-to-t transitions (1), the more difficult a-to-g and t-to-c transitions (2), and all transversions (3).
Figure. 5. Replication of wild-type and mutant HIV-1 LAI molecular clones.
Figure. 6. Replication of wild-type and mutant HIV-1 LAI molecular clones.
Figure. 7. Mutations in gp41e under T20 therapy and sequence variation in natural HIV-1 isolates. The GIV and SNY motifs in the HR1 and HR2 domains are boxed. The upper half of this figure tabulates the 91 sequences observed during T20 therapy, and the number of appearances is listed. The lower half of the figure tabulates natural sequence variation in the two motifs. A total of 338 HIV-1 subtype B sequences were obtained from the Los Alamos database (http://www.hiv.lanl.gov), with 338 HR1 and 332 HR2 sequences. The residues flanking the GIV and SNY motifs are not included for this set because of extensive sequence variation among the natural HIV-1 isolates. The NYT triplet that overlaps the SNY motif encodes a glycosylation site that is lost in some of the HR2 mutants (Perrin *et al*., 1998;Johnson *et al*., 2001;Lee *et al*., 1992;Dedera *et al*., 1992;Dash *et al*., 1994).
Figure. 8. Model for T20-dependency.

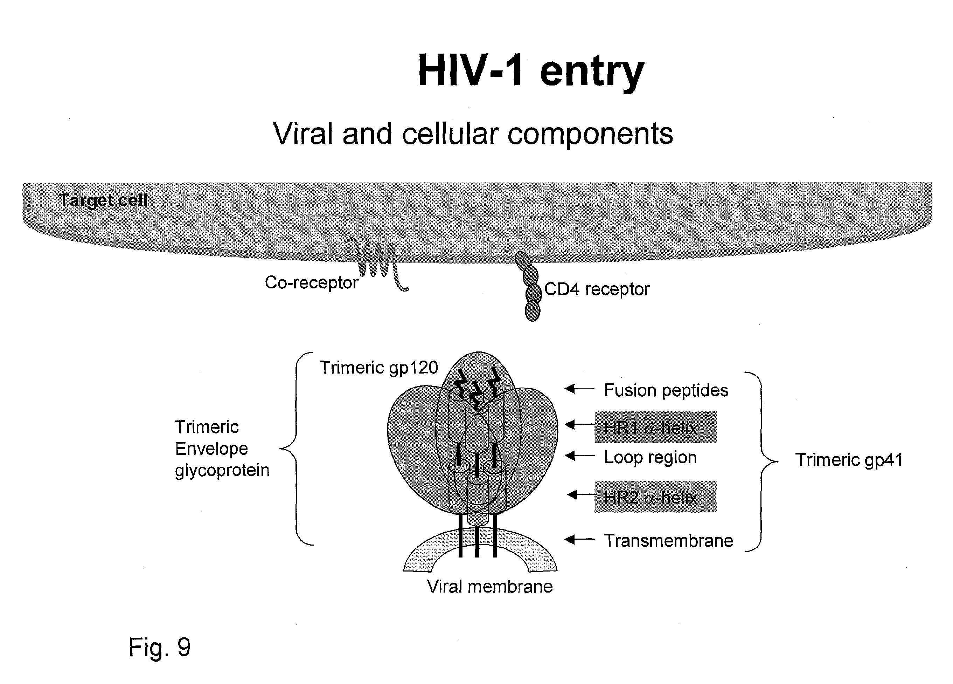

Figure 9. Model for wild type HIV entry into host cell.

### References

Back,N.K.T., Nijhuis,M., Keulen,W., Boucher,C.A.B., Oude Essink,B.B., van Kuilenburg,A.B.P., Van Gennip,A.H., and Berkhout,B. (1996). Reduced replication of 3TC-resistant HIV-1 variants in primary cells due to a processivity defect of the reverse transcriptase enzyme. *EMBO J.*, **15**, 4040-4049.
Barnett,J.M., Cadman,A., Burrell,F.M., Madar,S.H., Lewis,A.P., Tisdale,M., and Bethell,R. (1999). In vitro selection and characterisation of influenza B/Beijing/1/87 isolates with altered susceptibility to zanamivir. *Virol*., **265**, 286-295.
Bentz,J. (2000). Membrane fusion mediated by coiled coils: a hypothesis. *Biophys*. *J.,* **78**, 886-900.
Berkhout,B., Das,A.T., and Beerens,N. (2001). HIV-1 RNA editing, hypermutation and error-prone reverse transcription. *Science,* **292**, 7.
Berkhout,B. (1999). HIV-1 evolution under pressure of protease inhibitors, climbing the stairs of viral fitness. *J. Biomed. Sci.,* **6**, 298-305.
Bock,C.T., Tillmann,H.L., Torresi,J., Klempnauer,J., Locarnini,S., Manns,M.P., and Trautwein,C. (2002). Selection of hepatitis B virus polymerase mutants with enhanced replication by lamivudine treatment after liver transplantation. *Gastroenterology,* **122**, 264-273.
Boom,R., Sol,C.J.A., Salimans,M.M.M., Jansen,C.L., Wertheim-van Dillen,P.M.E., and Van der Noordaa,J. (1990). A rapid and simple method for purification of nucleic acids. *J. Clin. Microbiol.,* **28**, 495-503.
Boucher,C.A.B., Keulen,W., van Bommel,T., Nijhuis,M., de Jong,D., De Jong,M.D., Schipper,P., and Back,N.K.T. (1996). Human immunodeficiency virus type 1 drug susceptibility determination by using recombinant viruses generated from patient sera tested in a cell-killing assay. *Antim*. *Ag. Chemoth.,* **40**, 2404-2409.
Chan,D.C. and Kim,P.S. (1998). HIV entry and its inhibition. *Cell*, **93**, 681-684.
Church,J.A., Cunningham,C., Hughes,M., Palumbo,P., Mofenson,L.M., Delora,P., Smith,E., Wiznia,A., Purdue,L., Hawkins,E., and Sista,P. (2002). Safety and antiretroviral activity of chronic subcutaneous administration of T-20 in human immunodeficiency virus 1-infected children. *Pediatr. Infect. Dis. J.,* **21**, 653-659.
Cohen,C.J., Dusek,A., Green,J., Johns,E.L., Nelson,E., and Recny,M.A. (2002). Long-term treatment with subcutaneous T-20, a fusion inhibitor, in HIV-infected patients: patient satisfaction and impact on activities of daily living. *AIDS Patient. Care STDS.,* **16**, 327-335.
Cooley,L.A. and Lewin,S.R. (2003). HIV-1 cell entry and advances in viral entry inhibitor therapy. *J Clin Virol,* **26**, 121-132.
D'Souza,M.P., Cairns,J.S., and Plaeger,S.F. (2000). Current evidence and future directions for targeting HIV entry: therapeutic and prophylactic strategies. *JAMA*, **284**, 215-222.
Das,A.T., Klaver,B., and Berkhout,B. (1999). A hairpin structure in the R region of the Human Immunodeficiency Virus type 1 RNA genome is instrumental in polyadenylation site selection. *J. Virol*., **73**, 81-91.
Dash,B., McIntosh,A., Barrett,W., and Daniels,R. (1994). Deletion of a single N-linked glycosylation site from the transmembrane envelope protein of human immunodeficiency virus type 1 stops cleavage and transport of gp 160 preventing env-mediated fusion. *J. Gen. Virol*., **75 ( Pt 6**), 1389-1397.
De Clercq,E. (2002). New developments in anti-HIV chemotherapy. *Biochimica et Biophysica Acta (BBA) - Molecular Basis of Disease,* **1587**, 258-275.
Dedera,D.A., Gu,R.L., and Ratner,L. (1992). Role of asparagine-linked glycosylation in human immunodeficiency virus type 1 transmembrane envelope function. *Virol*., **187**, 377-382.
Doms,R.W. and Moore,J.P. (2000). HIV-1 membrane fusion: targets of opportunity. *J*. *Cell Biol.,* **151**, F9-14.
Doms,R.W. and Trono,D. (2000). The plasma membrane as a combat zone in the HIV battlefield. *Genes Dev.,* **14**, 2677-2688.
Dong,X.N., Xiao,Y., Dierich,M.P., and Chen,Y.H. (2001). N- and C-domains of HIV-1 gp41: mutation, structure and functions. *Immunol. Lett*., **75**, 215-220.
Doyon,L., Croteau,G., Thibeault,D., Poulin,F., Pilote,L., and Lamarre,D. (1996). Second locus involved in human immunodeficiency virus type 1 resistance to protease inhibitors. *J. Virol.,* **70**, 3763-3769.
Eckert,D.M. and Kim,P.S. (2001). Mechanisms of viral membrane fusion and its inhibition. *Annu. Rev. Biochem.,* **70**, 777-810.
Eckert,D.M., Malashkevich,V.N., Hong,L.H., Carr,P.A., and Kim,P.S. (1999). Inhibiting HIV-1 entry: discovery of D-peptide inhibitors that target the gp41 coiled-coil pocket. *Cell*, **99**, 103-115.
Furuta,R.A., Wild,C.T., Weng,Y., and Weiss,C.D. (1998). Capture of an early fusion-active conformation of HIV-1 gp41. *Nat. Struct. Biol.,* **5**, 276-279.
Hanna,S.L., Yang,C., Owen,S.M., and Lal,R.B. (2002). Resistance mutation in HIV entry inhibitors. AIDS, 16, 1603-1608.
Johnson,W.E., Sauvron,J.M., and Desrosiers,R.C. (2001). Conserved, N-linked carbohydrates of human immunodeficiency virus type 1 gp41 are largely dispensable for viral replication. *J*. *Virol*., **75**, 11426-11436.
Keulen,W., Back,N.K.T., van Wijk,A., Boucher,C.A.B., and Berkhout,B. (1997). Initial appearance of the 184Ile variant in lamivudine-treated patients is caused by the mutational bias of the human immunodeficiency virus type 1 Reverse Transcriptase. *J*. *Virol.,* **71**, 3346-3350.
Keulen,W., Boucher,C., and Berkhout,B. (1996). Nucleotide substitution patterns can predict the requirements for drug-resistance of HIV-1 proteins. *Antiviral Res.,* **31**, 45-57.
Kilby,J.M., Hopkins,S., Venetta,T.M., DiMassimo,B., Cloud,G.A., Lee,J.Y., Alldredge,L., Hunter,E., Lambert,D., Bolognesi,D., Matthews,T., Johnson,M.R., Nowak,M.A., Shaw,G.M., and Saag,M.S. (1998). Potent suppression of HIV-1 replication in humans by T-20, a peptide inhibitor of gp41-mediated virus entry. *Nat*. *Med.,* **4**, 1302-1307.
Kilby,J.M., Lalezari,J.P., Eron,J.J., Carlson,M., Cohen,C., Arduino,R.C., Goodgame,J.C., Gallant,J.E., Volberding,P., Murphy,R.L., Valentine,F., Saag,M.S., Nelson,E.L., Sista,P.R., and Dusek,A. (2002). The safety, plasma pharmacokinetics, and antiviral activity of subcutaneous enfuvirtide (T-20), a peptide inhibitor of gp41-mediated virus fusion, in HIV-infected adults. *AIDS Res. Hum. Retroviruses,* **18**, 685-693.
Lalezari,J.P., Eron,J.J., Carlson,M., Cohen,C., DeJesus,E., Arduino,R.C., Gallant,J.E., Volberding,P., Murphy,R.L., Valentine,F., Nelson,E.L., Sista,P.R., Dusek,A., and Kilby,J.M. (2003a). A phase II clinical study of the long-term safety and antiviral activity of enfuvirtide-based antiretroviral therapy. *AIDS,* **17**, 691-698.
Lalezari,J.P., Henry,K., O'Hearn,M., Montaner,J.S., Piliero,P.J., Trottier,B., Walmsley,S., Cohen,C., Kuritzkes,D.R., Eron,J.J., Jr., Chung,J., DeMasi,R., Donatacci,L., Drobnes,C., Delehanty,J., and Salgo,M. (2003b). Enfuvirtide, an HIV-1 Fusion Inhibitor, for Drug-Resistant HIV Infection in North and South America. *N Engl J Med.*
Layne,S.P., Merges,M.J., Dembo,M., Spouge,J.L., Conley,S.R., Moore,J.P., Raina,J.L., Renz,H., Gelderblom,H.R., and Nara,P.L. (1992). Factors underlying spontaneous inactivation and susceptibility to neutralization of human immunodeficiency virus. *Virol*., **189**, 695-**714**.
Lee,W.R., Yu,X.F., Syu,W.J., Essex,M., and Lee,T.H. (1992). Mutational analysis of conserved N-linked glycosylation sites of human immunodeficiency virus type 1 gp41. *J. Virol*., **66**, 1799-1803.
Little,S.J., Holte,S., Routy,J.P., Daar,E.S., Markowitz,M., Collier,A.C., Koup,R.A., Mellors,J.W., Connick,E., Conway,B., Kilby,M., Wang,L., Whitcomb,J.M., Hellmann,N.S., and Richman,D.D. (2002). Antiretroviral-drug resistance among patients recently infected with HIV. *N. Engl. J. Med.,* **347**, 385-394.
Mammano,F., Petit,C., and Clavel,F. (1998). Resistance-associated loss of viral fitness in human immunodeficiency virus type 1: phenotypic analysis of Protease and gag coevolution in protease inhibitor-treated patients. *J. Virol.*, **72**, 7632-7637.
Marzio,G., Verhoef,K., Vink,M., and Berkhout,B. (2001). *In vitro* evolution of a highly replicating, doxycycline-dependent HIV for applications in vaccine studies. *Proc. Natl. Acad. Sci. USA*, **98**, 6342-6347.
Matsuoka-Aizawa,S., Sato,H., Hachiya,A., Tsuchiya,K., Takebe,Y., Gatanaga,H., Kimura,S., and Oka,S. (2003). Isolation and molecular characterization of a nelfinavir (NFV)-resistant human immunodeficiency virus type 1 that exhibits NFV-dependent enhancement of replication. *J Virol*, **77**, 318-327.
Meis,R.J. and Condit,R.C. (1991). Genetic and molecular biological characterization of a vaccinia virus gene which renders the virus dependent on isatin-beta-thiosemicarbazone (IBT). *Virol.*, **182**, 442-454.
Melikyan,G.B., Markosyan,R.M., Hemmati,H., Delmedico,M.K., Lambert,D.M., and Cohen,F.S. (2000). Evidence that the transition of HIV-1 gp41 into a six-helix bundle, not the bundle configuration, induces membrane fusion. *J. Cell Biol*., **151**, 413-423.
Menzo,S., Monachetti,A., Balotta,C., Corvasce,S., Rusconi,S., Paolucci,S., Baldanti,F., Bagnarelli,P., and Clementi,M. (2003). Processivity and drug-dependence of HIV-1 protease: determinants of viral fitness in variants resistant to protease inhibitors. *AIDS*, **17**, 663-671.
Moore,J.P. and Stevenson,M. (2000). New targets for inhibitors of HIV-1 replication. *Nat. Rev*. *Mol*. *Cell Biol.,* **1**, 40-49.
Peden,K., Emerman,M., and Montagnier,L. (1991). Changes in growth properties on passage in tissue culture of viruses derived from infectious molecular clones of HIV-1_{LAI}, HIV-1_{MAL}, and HIV-1_{ELI}. *Virol*., **185**, 661-672.
Perrin,C., Fenouillet,E., and Jones,I.M. (1998). Role of gp41 glycosylation sites in the biological activity of human immunodeficiency virus type 1 envelope glycoprotein. *Virol.*, **242**, 338-345.
Reeves,J.D., Gallo,S.A., Ahmad,N., Miamidian,J.L., Harvey,P.E., Sharron,M., Pohlmann,S., Sfakianos,J.N., Derdeyn,C.A., Blumenthal,R., Hunter,E., and Doms,R.W. (2002). Sensitivity of HIV-1 to entry inhibitors correlates with envelope/coreceptor affinity, receptor density, and fusion kinetics. *Proc Natl Acad Sci U S A*.
Rimsky,L.T., Shugars,D.C., and Matthews,T.J. (1998). Determinants of human immunodeficiency virus type 1 resistance to gp41- derived inhibitory peptides. *J. Virol*., **72**, 986-993.
Roos,J.W., Maughan,M.F., Liao,Z., Hildreth,J.E., and Clements,J.E. (2000). LuSIV cells: a reporter cell line for the detection and quantitation of a single cycle of HIV and SIV replication. *Virol*., **273**, 307-315.
Tolskaya,E.A., Romanova,L.I., Kolesnikova,M.S., Gmyl,A.P., Gorbalenya,A.E., and Agol,V.I. (1994). Genetic studies on the poliovirus 2C protein, an NTPase. A plausible mechanism of guanidine effect on the 2C function and evidence for the importance of 2C oligomerization. *J Mol*. *Biol,* **236**, 1310-1323.
Verhoef,K., Marzio,G., Hillen,W., Bujard,H., and Berkhout,B. (2001). Strict control of human immunodeficiency virus type 1 replication by a genetic switch: Tet for Tat. *J. Virol.,* **75**, 979-987.
Wang,W., Lee,W.M., Mosser,A.G., and Rueckert,R.R. (1998). WIN 52035-dependent human rhinovirus 16: assembly deficiency caused by mutations near the canyon surface. *J Virol,* **72**, 1210-1218.
Wei,X., Decker,J.M., Liu,H., Zhang,Z., Arani,R.B., Kilby,J.M., Saag,M.S., Wu,X., Shaw,G.M., and Kappes,J.C. (2002). Emergence of resistant human immunodeficiency virus type 1 in patients receiving fusion inhibitor (T-20) monotherapy. *Antimicrob. Agents Chemother.,* **46**, 1896-1905.
Weissenhorn,W., Dessen,A., Harrison,S.C., Skehel,J.J., and Wiley,D.C. (1997). Atomic structure of the ectodomain from HIV-1 gp41. *Nature*, **387**, 426-430.
Wild,C., Dubay,J.W., Greenwell,T., Baird,T., Jr., Oas,T.G., McDanal,C., Hunter,E., and Matthews,T. (1994a). Propensity for a leucine zipper-like domain of human immunodeficiency virus type 1 gp41 to form oligomers correlates with a role in virus- induced fusion rather than assembly of the glycoprotein complex. *Proc. Natl*. *Acad. Sci. U. S. A*, **91**, 12676-12680.
Wild,C.T., Shugars,D.C., Greenwell,T.K., McDanal,C.B., and Matthews,T.J. (1994b). Peptides corresponding to a predictive alpha-helical domain of human immunodeficiency virus type 1 gp41 are potent inhibitors of virus infection. *Proc. Natl*. *Acad. Sci. U. S. A*, **91**, 9770-9774.
Xu,L., Hue,S., Taylor,S., Ratcliffe,D., Workman,J.A., Jackson,S., Cane,P.A., and Pillay,D. (2002). Minimal variation in T-20 binding domain of different HIV-1 subtypes from antiretroviral-naive and -experienced patients. *AIDS*, **16**, 1684-1686.
Zollner,B., Feucht,H.H., Schroter,M., Schafer,P., Plettenberg,A., Stoehr,A., and Laufs,R. (2001). Primary genotypic resistance of HIV-1 to the fusion inhibitor T-20 in long-term infected patients. *AIDS,* **15**, 935-936.
Baba TW, Liska V, Khimani AH, Ray NB, Dailey PJ, Penninck D, Bronson R, Greene MF, McClure HM, Martin LN, et al. 1999. Live attenuated, multiply deleted simian immunodeficiency virus causes AIDS in infant and adult macaques. *Nature Medicine 5*:194-203.
Berkhout B. 1999. Multiple biological roles associated with the repeat (R) region of the HIV-1 RNA genome. *Adv*.*Pharmacol*. *in press:*
Berkhout B, Das AT. 1999. Functional analysis of RNA signals in the HIV-1 genome by forced evolution. In: Barciszewski J, Clark BFC, eds. *RNA biochemistry and biotechnology.* Dordrecht, the Netherlands.: Kluwer Academic Publishers; pp. in press
Berkhout B, Verhoef K, van Wamel JLB, Back B. 1999. Genetic instability of live-attenuated HIV-1 vaccine strains. *J*. *Virol*. *73*:1138-45.
Klaver B, Berkhout B. 1994. Evolution of a disrupted TAR RNA hairpin structure in the HIV-1 virus. *EMBO J*. *13*:2650-9.
Klaver B, Berkhout B. 1994. Premature strand transfer by the HIV-1 reverse transcriptase during strong-stop DNA synthesis. *Nucleic Acids Res. 22*:137-44.
Zwick MB, Labrijn AF, Wang M, Spenlehauer C, Saphire EO, Binley JM, Moore JP, Stiegler G, Katinger H, Burton DR, Parren PW. Broadly neutralizing antibodies targeted to the membrane-proximal external region of human immunodeficiency virus type 1 glycoprotein gp41. J Virol. 2001 Nov;75(22):10892-905.
Murphy F. Virus Taxonomy. In: Fields Virology, third edition. 1996. Fields BN, Knipe DM, Howley PM.
   Lippincot - Raven publishers, Philadelphia, pages 24-27

## Claims

1. A method for rendering a virus dependent on an inducing agent for viral entry by means of a pairing of two domains (HR1 and HR2) of an envelope glycoprotein into a host cell, wherein said inducing agent is capable of interfering in said pairing, comprising increasing the affinity between a HR1 domain and a HR2 domain of said envelope glycoprotein of said virus to such extent that said pairing occurs without the presence of a host cell (a premature switch) when said inducing agent is essentially not present.

2. A method according to claim 1, wherein said affinity is increased by inducing a mutation in a nucleic acid of said virus encoding at least part of said HR1 domain.

3. A method according to claim 1 or 2, wherein said affinity is increased by inducing a mutation in a nucleic acid of said virus encoding at least part of said HR2 domain.

4. A method according to any one of claims 1-3, comprising inducing at least one mutation in a nucleic acid of said virus encoding a GIV motif of said HR1 domain.

5. A method according to claim 1 or 2, comprising inducing at least one mutation in a nucleic acid of said virus encoding a SNY motif of said HR2 domain.

6. A method according to any one of claims 1-5, comprising inducing at least one mutation in a nucleic acid of said virus encoding a GIV motif of said HR1 domain and a mutation in a nucleic acid of said virus encoding a SNY motif of said HR2 domain.

7. A method according to any one of claims 2-4, wherein said mutation in said GIV motif comprises a GIG, GIW and/or GIA mutation.

8. A method according to any one of claims 3-5, wherein said mutation in said SNY motif comprises a SKY mutation.

9. A method according to any one of claims 4-6, wherein said mutation in said GIV motif is a GIA mutation and said mutation in said SNY motif is a SKY mutation.

10. A method according to any one of claims 1-9, wherein said inducing agent comprises a peptide fusion inhibitor or a chaotrophic agent.

11. A method according to any one of claims 1-10, wherein said peptide fusion inhibitor comprises T20 or a functional part, derivative and/or analogue thereof.

12. A virus dependent on an inducing agent for viral entry obtainable by a method according to any one of claims 1-11.

13. A virus according to claim 12 which is a human immunodeficiency virus.

14. A virus according to claim 12 or 13 which is an attenuated virus.

15. A viral replicon comprising a nucleic acid sequence with a mutation in a region encoding a HR1 domain and/or HR2 domain of an envelope glycoprotein, resulting in an increased affinity between said HR1 domain and said HR2 domain to such extent that pairing of said HR1 and HR2 domains occurs without the presence of a host cell when an inducing agent is essentially not present.

16. A viral replicon according to claim 15, wherein at least part of said nucleic acid sequence comprises RNA.

17. A virus according to any one of claims 12-14 or a viral replicon according to claim 15 or 16 which is derived from a human immunodeficiency virus.

18. A virus or viral replicon according to any one of claims 12-17 which is derived from an infectious human immunodeficiency virus clone.

19. A virus or viral replicon according to any one of claims 12-18, further comprising at least one inducible repressor and/or activator for replication.

20. A virus or viral replicon according to claim 19, wherein said inducible repressor and/or activator comprises a Tet operon or a functional equivalent thereof, wherein at least one functional part, preferably an rtTA gene, of said inducible repressor and/or activator is inserted into the nef gene, wherein at least one NF-kb element has been deleted, and/or wherein at least one LTR is modified to avoid reversion to wild type virus.

21. A virus or viral replicon according to claim 19 or 20 which further comprises an inactivated TAR element, an inactivated Tat element, and/or at least one tet0 motif in at least one functional LTR.

22. A virus or viral replicon according to any one of claims 12-21 which further comprises a suicide gene.

23. A method for producing a virus dependent on an inducing agent for viral entry, comprising:
- providing a permissive cell with a virus and/or a viral replicon according to any one of claims 12-22;
- culturing said cell; and
- harvesting said dependent virus from said culture.

24. A method according to claim 23, wherein said cell is cultured in the presence of said inducing agent.

25. A method according to claim 23 or 24 wherein said dependent virus is a human immunodeficiency virus.

26. A method according to any one of claims 23-25, wherein said virus is an attenuated virus.

27. A method according to any one of claims 23-26, wherein said inducing agent comprises a peptide fusion inhibitor or a chaotrophic agent.

28. A method according to any one of claims 23-27, wherein said peptide fusion inhibitor comprises T20 or a functional part, derivative and/or analogue thereof.

29. A vaccine comprising a virus and/or a viral replicon according to any one of claims 12-22.

30. A kit of parts comprising a virus and/or a viral replicon according to any one of claims 12-22 and an amount of inducing agent.

31. A kit of parts according to claim 29 wherein said inducing agent is provided separately.

32. A vaccine according to claim 29 or a kit of parts according to claim 30 or 31, wherein said replicon and/or said virus is derived from a human immunodeficiency virus.

33. A method for the controlled replication of a virus and/or a viral replicon comprising providing a permissive cell with a virus and/or replicon according to any one of claims 12-22, culturing said cell in the presence of said inducing agent and manipulating the amount of inducing agent present.

34. A method for the prophylaxis of AIDS, comprising administering a vaccine according to claim 29 or a kit of parts according to claim 30 or 31 to a patient and allowing for viral entry for a limited time by providing said inducing agent.

35. A method for modifying a virus and/or viral replicon according to anyone of claims 12-22, comprising:
- generating a virus and/or viral replicon according to any one of claims 12-22,
- providing cells, permissive for replication of said virus and/or replicon, with said virus and/or replicon,
- culturing said cells under conditions that allow replication of said virus and/or replicon,
- obtaining replicated virus and/or replicon from said culture.

36. A viral nucleic acid sequence comprising a mutation in a region encoding a HR1 domain and/or HR2 domain of an envelope glycoprotein resulting in an increased affinity between said HR1 domain and said HR2 domain to such extent that pairing of said HR1 and HR2 domains occurs when an inducing agent is essentially not present.

37. A cell comprising a replicon according to anyone of claims 15-22.

38. A cell comprising a nucleic acid according to claim 36.

39. An isolated or recombinant proteinaceous molecule capable of specifically binding a virus according to any one of claims 12-22.
